(19) Europäisches Patentamt
European Patent Office
Office européen des brevets

(11) Veröffentlichungsnummer: **0 049 417**
**A2**

(12) # EUROPÄISCHE PATENTANMELDUNG

(21) Anmeldenummer: **81107512.6**

(22) Anmeldetag: **22.09.81**

(51) Int. Cl.³: **C 07 F 7/18**
C 07 C 62/02, C 07 C 62/32
C 07 C 51/00

(30) Priorität: **02.10.80 DE 3037302**

(43) Veröffentlichungstag der Anmeldung:
**14.04.82 Patentblatt 82/15**

(84) Benannte Vertragsstaaten:
**BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Heine, Hans-Georg, Dr.**
**Am Heckerhof 14**
**D-4150 Krefeld 1(DE)**

(72) Erfinder: **Knops, Hans-Joachim, Dr.**
**Claudiusweg 3**
**D-5600 Wuppertal 1(DE)**

(72) Erfinder: **Priesnitz, Uwe, Dr.**
**Severinstrasse 58**
**D-5650 Solingen 1(DE)**

(54) **Bissilylierte 1-Hydroxycyclopropancarbonsäuren, Verfahren zu ihrer Herstellung sowie ihre Verwendung als Zwischenprodukte.**

(57) Die Erfindung betrifft neue bissilylierte 1-Hydroxycyclopropancarbonsäuren der allgemeinen Formel (I)

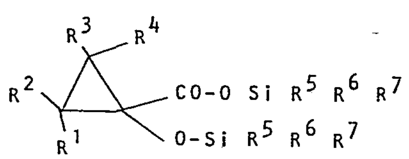

in der die Reste $R^1$ mit $R^7$ die in der Beschreibung angegebene Bedeutung haben,
sowie ein Verfahren zu ihrer Herstellung.

Die erfindungsgemäßen Verbindungen werden durch Oxidation mit Sauerstoff aus geeigneten 1,2-Bis-trialkylsilsiloxy-cyclo-but-1-enen hergestellt.

Die erfindungsgemäßen Verbindungen werden zur Herstellung von 1-Hydroxycyclopropancarbonsäuren verwendet.

EP 0 049 417 A2

0049417



BAYER AKTIENGESELLSCHAFT 5090 Leverkusen, Bayerwerk
Zentralbereich Slr/W
Patente, Marken und Lizenzen Ia/ZP

# Bissilylierte 1-Hydroxycyclopropancarbonsäuren, Verfahren zu ihrer Herstellung sowie ihre Verwendung als Zwischenprodukte

Die vorliegende Erfindung betrifft neue bissilylierte 1-Hydroxycyclopropancarbonsäuren, die als Zwischenprodukte für die Synthese von 1-Hydroxycyclopropancarbonsäuren verwendet werden können , sowie ein Verfahren zu deren Herstellung.

Es wurden als neue Verbindungen die bissilylierten 1-Hydroxycyclopropancarbonsäuren der allgemeinen Formel

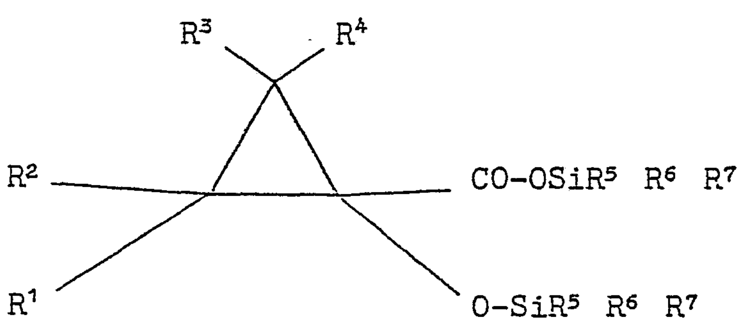

(I)

in welcher

$R^1$, $R^2$, $R^3$ und $R^4$ gleich oder verschieden sind und für Wasserstoff oder Alkyl stehen, außerdem können $R^2$ und $R^3$ auch gemeinsam für eine gesättigte oder ungesättigte 3- bis 5-gliedrige Methylenbrücke stehen, und

$R^5$, $R^6$ und $R^7$ gleich oder verschieden sind und für Alkyl stehen,

gefunden.

Die erfindungsgemäßen Verbindungen der Formel (I) können gegebenenfalls in geometrischen (cis,trans) und optischen Isomeren vorkommen; vorzugsweise fallen sie als Isomerengemische an.

Weiterhin wurde gefunden, daß man die bissilylierten 1-Hydroxycyclopropancarbonsäuren der Formel (I) erhält, wenn man 1,2-Bis-trialkylsiloxy-cyclo-but-1-ene der Formel

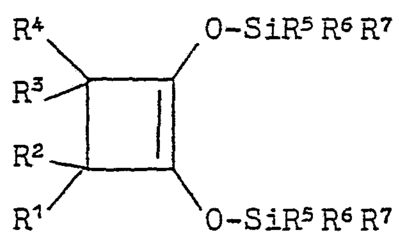

(II)

in welcher

$R^1$ bis $R^7$ die oben angegebene Bedeutung haben,

mit Sauerstoff als Oxidationsmittel, gegebenenfalls in Gegenwart eines inerten organischen Verdünnungsmittels sowie gegebenenfalls in Gegenwart eines Katalysators, umsetzt.

Die erfindungsgemäßen Stoffe sind durch die Formel (I) allgemein definiert. In dieser Formel sind $R^1$, $R^2$, $R^3$ und $R^4$ gleich oder verschieden und stehen vorzugsweise für Wasserstoff oder Alkyl mit 1 bis 4 Kohlenstoffatomen. $R^2$ und $R^3$ stehen außerdem gemeinsam vorzugsweise für eine gegebenenfalls ungesättigte Tetramethylenbrücke. $R^5$, $R^6$ und $R^7$ sind gleich oder verschieden und stehen vorzugsweise für Alkyl mit 1 bis 4 Kohlenstoffatomen. Als Beispiele seien genannt:

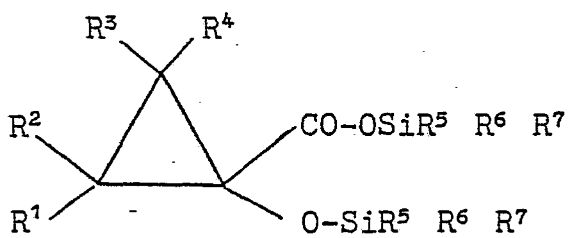

(I)

| $R^1$ | $R^2$ | $R^3$ | $R^4$ | $R^5$ | $R^6$ | $R^7$ |
|---|---|---|---|---|---|---|
| H | H | H | H | $CH_3$ | $CH_3$ | $CH_3$ |
| $CH_3$ | $CH_3$ | H | H | $CH_3$ | $CH_3$ | $CH_3$ |
| $CH_3$ | $CH_3$ | $CH_3$ | $CH_3$ | $CH_3$ | $CH_3$ | $CH_3$ |
| $CH_3$ | H | H | H | $CH_3$ | $CH_3$ | $CH_3$ |
| $C_2H_5$ | H | H | H | $CH_3$ | $CH_3$ | $CH_3$ |
| i-$C_3H_7$ | H | H | H | $CH_3$ | $CH_3$ | $CH_3$ |
| t-$C_4H_9$ | H | H | H | $CH_3$ | $CH_3$ | $CH_3$ |
| n-$C_3H_7$ | H | H | H | $CH_3$ | $CH_3$ | $CH_3$ |
| n-$C_4H_9$ | H | H | H | $CH_3$ | $CH_3$ | $CH_3$ |
| i-$C_4H_9$ | H | H | H | $CH_3$ | $CH_3$ | $CH_3$ |
| H | $-CH_2CH_2CH_2CH_2-$ | | H | $CH_3$ | $CH_3$ | $CH_3$ |
| H | $-CH_2-CH=CH-CH_2-$ | | H | $CH_3$ | $CH_3$ | $CH_3$ |

Verwendet man beispielsweise 1,2-Bis-trimethylsiloxy-cyclobut-1-ene und Luft als Ausgangsstoffe, so kann der Reaktionsablauf durch das folgende Formelschema wiedergegeben werden:

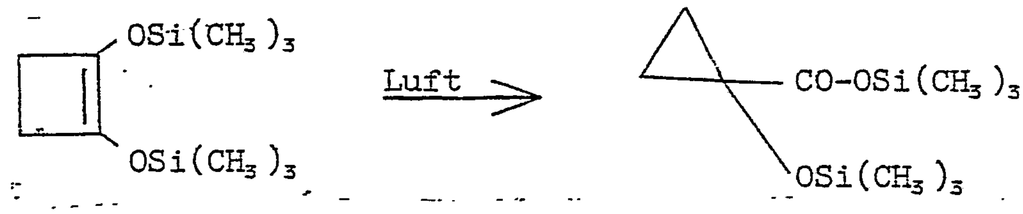


Es ist ausgesprochen überraschend, daß unter den angegebenen Reaktionsbedingungen beide Silylgruppen erhalten bleiben und unter Aufnahme von formal einem Sauerstoffatom die Ringverengung eintritt.

Die für das erfindungsgemäße Verfahren als Ausgangsstoffe zu verwendenden 1,2-Bis-trialkylsiloxy-cyclobut-1-ene sind durch die Formel (II) allgemein definiert. In dieser Formel stehen $\underline{R}^1$ bis $\underline{R}^7$ vorzugsweise für diejenigen Reste, die bereits bei der Beschreibung der erfindungsgemäßen Stoffe der Formel (I) vorzugsweise für diese Substituenten genannt wurden.

Die 1,2-Bis-trialkylsiloxy-cyclobut-1-ene der Formel (II) sind bekannt (vergleiche z.B. Synthesis 1971, 236ff), bzw. können sie nach den dort angegebenen Verfahren durch Acyloinkondensation der Bernsteinsäureester in Gegenwart von Trialkylsilanen und Natrium erhalten werden.

Der für das erfindungsgemäße Verfahren als Oxidationsmittel zu verwendende Sauerstoff wird vorzugsweise in Form von Luft eingesetzt. Es ist auch möglich, mit durch Lichtanregung in Gegenwart eines Sensibilisators erzeugtem Singulett-Sauerstoff zu arbeiten.

Die erfindungsgemäße Umsetzung wird gegebenenfalls in Gegenwart eines inerten organischen Verdünnungsmittels durchgeführt. Hierzu gehören vorzugsweise aromatische Kohlenwasserstoffe, wie Toluol und Xylol; Ether wie Diethylether; oder chlorierte Kohlenwasserstoffe, wie Chlorbenzol oder Tetrachlorkohlenstoff.
Es sind aber grundsätzlich auch andere inerte organische Lösungsmittel möglich, wie beispielsweise Nitrile und Ester.

Die erfindungsgemäße Umsetzung kann gegebenenfalls in Gegenwart eines Katalysators durchgeführt werden. Man kann alle üblicherweise für die Luftoxidation verwendbaren Katalysatoren einsetzen, wie beispielsweise Oxide und Salze von Uebergangsmetallen. Es kann aber auch in Gegenwart von Licht gearbeitet werden.

Die Reaktionstemperaturen können bei der erfindungsgemäßen Umsetzung in einem größeren Bereich variiert werden. Im allgemeinen arbeitet man zwischen 0 und 150°C, vorzugsweise zwischen 20 und 120°C.

Die Verbindungen der Formel (I) können in üblicher Weise isoliert werden. Sie lassen sich aber auch direkt weiter zu den entsprechenden 1-Hydroxycyclopropancarbonsäuren der Formel

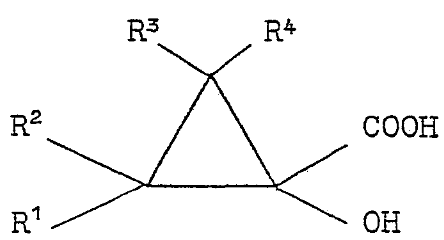

(III)

in welcher

$R^1$ bis $R^4$ die oben angegebene Bedeutung haben,

umsetzen, indem man in die Reaktionslösung trockenen, gasförmigen Halogenwasserstoff, wie insbesondere Chlorwasserstoff, einleitet. Dabei fallen die 1-Hydroxycyclopropancarbonsäuren als kristalline Feststoffe an, die abgesaugt und getrocknet werden.

Die neuen bissilylierten 1-Hydroxycyclopropancarbonsäuren der Formel (I) stellen somit, wie schon erwähnt, interessante Zwischenprodukte zur Herstellung von 1-Hydroxycyclopropancarbonsäuren dar, die auf diese Art und Weise in einer einfachen Eintopfreaktion rein und in guten Ausbeuten, auch im technischen Maßstab, erhalten werden können.

Die 1-Hydroxycyclopropancarbonsäuren der Formel (III) sind weitgehend bekannt (vergleiche z.B. Liebigs Ann. Chem. 1976, 463ff). Sie stellen allgemein interessante Zwischenprodukte dar, insbesondere auch für die Synthese von fungiziden Wirkstoffen.

So erhält man z.B. durch Umsetzung mit 3,5-Dihalogenphenylisocyanaten in Gegenwart eines inerten organischen Lösungsmittels, wie beispielsweise aromatische Kohlenwasserstoffe, und gegebenenfalls in Gegenwart einer Base, wie beispielsweise Triethylamin, bei Temperaturen zwischen 20 und 100°C die fungizid gut wirksamen Spiro-Derivate von 3-(3,5-Dihalogenphenyl)-oxazolidin-2,4-dionen der Formel

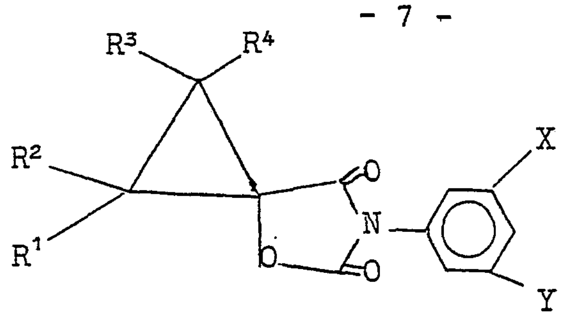

(IV)

in welcher

$R^1$ bis $R^4$ die oben angegebene Bedeutung haben und

X und Y für Halogen stehen

(vergleiche DE-OS 28 04 824 [LeA 18 673] und DE-OS 28 52 924 [LeA 19 330] ).

Beispiel 1

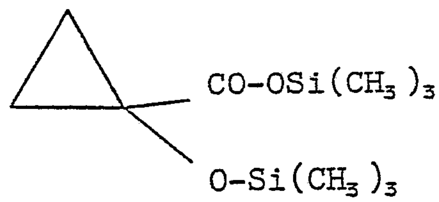

a) In 46,0 g (0,2 Mol) Cyclobuten-1,2-diol-bis-trimethylsilylether wird bei 120°C Badtemperatur unter Rühren getrocknete Luft eingeleitet. Der Umsatz wird NMRspektroskopisch verfolgt. Nach 6,5 Stunden läßt man abkühlen und destilliert fraktionierend an einer Ringspaltkolonne. Man erhält 33,7 g (68 % der Theorie) 1-Trimethylsiloxy-cyclopropancarbonsäure-trimethylsilylester vom Siedepunkt 78-80°C/17,5 mbar und vom Brechungsindex $n_D^{20}$= 1,422.

b) In 46,0 g (0,2 Mol) Cyclobuten-1,2-diol-bis-trimethylsilylether wird bei einer Temperatur von 20°C unter Rühren getrocknete Luft eingeleitet, bis vollständiger Umsatz erfolgt ist (72 Stunden). Das schwach gelb gefärbte Reaktionsprodukt wird an der Ringspaltkolonne destilliert. Man erhält 32,0 g (66 % der Theorie) 1-Trimethylsiloxycyclopropancarbonsäure-trimethylsilylester vom oben genannten Siedepunkt.

Folgeprodukt

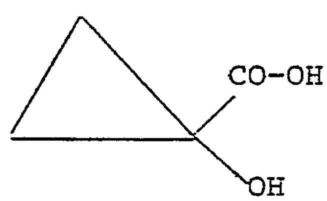

(Darstellung ausgehend von Cyclobuten-1,2-diol-bis-trimethylsilylether wie in Beispiel 1, wobei jedoch das Endprodukt gemäß Beispiel 1 nicht isoliert wird,

sondern unmittelbar zur 1-Hydroxy-cyclopropancarbonsäure gemäß obiger Formel weiter verarbeitet wird).

80g 1,2-Bis(trimethylsiloxy)-cyclobut-1-en werden mit 250 ml Toluol verdünnt und unter Rühren wird bei 90°C 18-24 Stunden Luft eingeleitet. Die Vollständigkeit der Umsetzung wird gaschromatographisch kontrolliert. Anschließend wird bei Raumtemperatur 2 bis 3 Stunden Chlorwasserstoff eingeleitet, das ausgefallene Kristallisat abgesaugt und aus Toluol umkristallisiert. Man erhält 26,7 g (75 % der Theorie, bezogen auf eingesetzten Silylether) 1-Hydroxy-cyclopropancarbonsäure vom Schmelzpunkt 102 - 105°C.

Beispiel 2

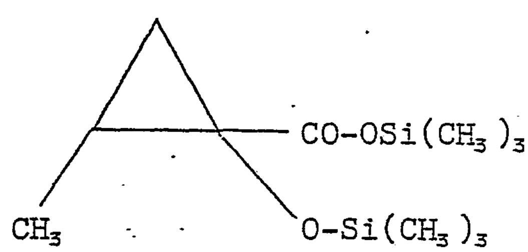

In 20,0g (0,082 Mol) 3-Methyl-1,2-bis(trimethylsiloxy)-cyclobut-1-en wird bei 100°C unter Rühren getrocknete Luft eingeleitet, bis vollständiger Umsatz erfolgt ist (4-6 Stunden). Nach Abkühlen auf 50°C destilliert man fraktionierend. Man erhält 14,2g (67 % der Theorie) 2-Methyl-1-trimethylsiloxy-cyclopropancarbonsäure-trimethylsilylester als Isomerengemisch vom Siedepunkt 90-97°C/17,5 mbar und vom Brechungsindex $n_D^{20}$ = 1,424.

Beispiel 3

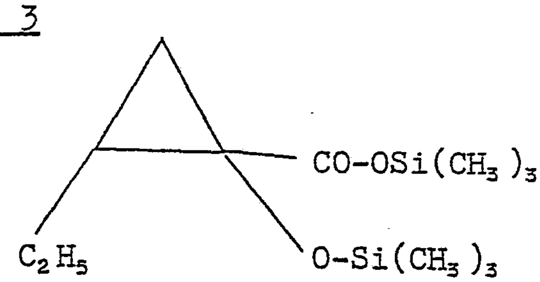

Entsprechend Beispiel 2 erhält man, ausgehend von 3-Ethyl-1,2-bis(trimethylsiloxy)-cyclobut-1-en in 75 %-iger Ausbeute 2-Ethyl-1-trimethylsiloxy-cyclopropan-carbonsäure-trimethylsilylester als Isomerengemisch vom Siedepunkt 48-57°C/ 0,4 mbar und vom Brechungsindex $n_D^{20}$ = 1,425.

Beispiel 4

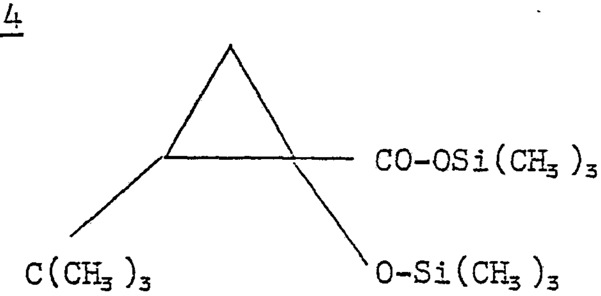

Entsprechend Beispiel 2 erhält man, ausgehend von 3-tert-Butyl-1,2-bis(trimethylsiloxy)-cyclobut-1-en in 57 %-iger Ausbeute (bezogen auf Umsatz) 2-tert-Butyl-1-trimethylsiloxycyclopropancarbonsäure-trimethylsilylester als Isomerengemisch vom Siedepunkt 48 - 57°C / 0,2 mbar und vom Brechungsindex $n_D^{20}$ = 1,4372.

Beispiel 5

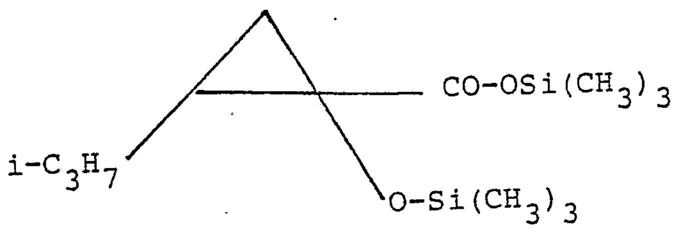

Entsprechend Beispiel 2 erhält man ausgehend von 3-Isopropyl-1,2-bis(Trimethylsiloxy)-cyclobut-1-en (0,1 mol) in 60 %-iger Ausbeute 2-Isopropyl-1-trimethylsiloxy-cyclopropancarbonsäure-trimethylsilylester vom Siedepunkt 100 - 111 °C/13 mbar und vom Brechungsindex $n_D^{20}$ = 1,4282.

Folgeprodukt

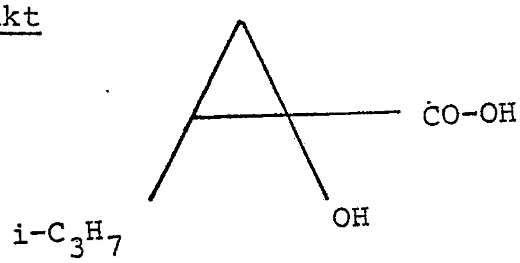

17,0 g ( 0,06 mol) 2-Isopropyl-1-trimethylsiloxy-cyclopropancarbonsäure-trimethylsilylester werden 2 h in 100 ml mit Chlorwasserstoff gesättigtem Toluol bei 30 °C gerührt. Anschließend leitet man 1 h trockenen Stickstoff durch die Lösung und filtriert den Niederschlag ab. Kristallisieren aus Xylol liefert 2-Isopropyl-1-hydroxy-cyclopropancarbonsäure (6,1 g) vom Schmelzpunkt 135 - 138 °C.

Beispiel 6

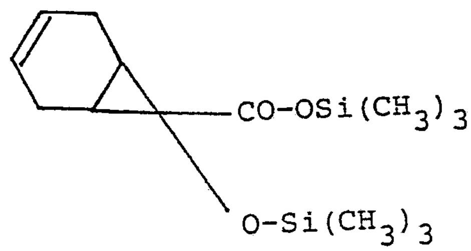

Entsprechend Beispiel 2 erhält man, ausgehend von 7,8-Bis-Trimethylsiloxy-bicyclo[4,2,0]octa-3,7-dien (0,1 mol) in 54 %-iger Ausbeute 7-Trimethylsiloxy-bicyclo[4,1,0]hept-3-en-7-carbonsäuretrimethylsilylester vom Siedepunkt 71 - 82 °C / 0,1 - 1,0 mbar und vom Brechungsindex $n_D^{20}$ = 1,4642.

Patentansprüche

1) Bissilylierte 1-Hydroxycyclopropancarbonsäuren der allgemeinen Formel

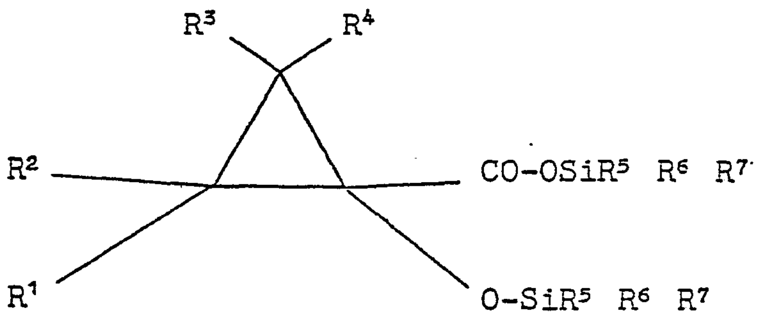

(I)

in welcher

$R^1$, $R^2$, $R^3$ und $R^4$ gleich oder verschieden sind und für Wasserstoff oder Alkyl stehen, außerdem können $R^2$ und $R^3$ auch gemeinsam für eine gesättigte oder ungesättigte 3- bis 5-gliedrige Methylenbrücke stehen, und

$R^5$, $R^6$ und $R^7$ gleich oder verschieden sind und für Alkyl stehen.

2) Verfahren zur Herstellung von bissilylierten 1-Hydroxycyclopropancarbonsäuren der allgemeinen Formel

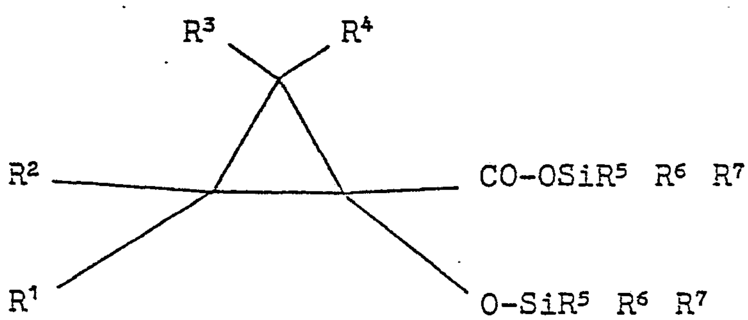

(I)

in welcher

$R^1$, $R^2$, $R^3$ und $R^4$ gleich oder verschieden sind und für Wasserstoff oder Alkyl stehen, außerdem können $R^2$ und $R^3$ auch gemeinsam für eine gesättigte oder ungesättigte 3- bis 5-gliedrige Methylenbrücke stehen, und

$R^5$, $R^6$ und $R^7$ gleich oder verschieden sind und für Alkyl stehen,

dadurch gekennzeichnet, daß man 1,2-Bis-trialkylsiloxy-cyclobut-1-ene der Formel

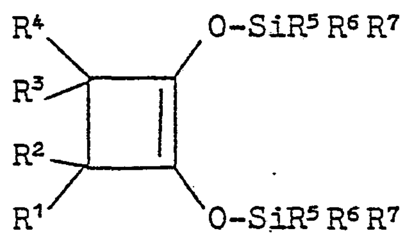

(II)

in welcher

$R^1$ bis $R^7$ die oben angegebene Bedeutung haben,

mit Sauerstoff als Oxidationsmittel, gegebenenfalls in Gegenwart eines inerten organischen Verdünnungsmittels sowie gegebenenfalls in Gegenwart eines Katalysators, umsetzt.

3) Verwendung von bissilylierten 1-Hydroxycyclopropancarbonsäuren gemäß Ansprüchen 1 und 2 als Zwischenprodukte zur Herstellung von 1-Hydroxycyclopropancarbonsäuren.

4) Verfahren zur Herstellung von 1-Hydroxycyclopropancarbonsäuren, dadurch gekennzeichnet, daß man zunächst die bissilylierten 1-Hydroxycyclopropancarbonsäuren gemäß Anspruch 2 herstellt und ohne deren Isolierung in die Reaktionslösung trockenen gasförmigen Halogenwasserstoff einleitet und die anfallenden 1-Hydroxycyclopropancarbonsäuren abtrennt.